# EUROPÄISCHE PATENTSCHRIFT

(11) **EP 1 841 471 B1**
(45) Veröffentlichungstag und Bekanntmachung des Hinweises auf die Patenterteilung: **28.07.2010**
(21) Anmeldenummer: 06777174.1
(22) Anmeldetag: 07.09.2006
(51) Int. Cl.: A61L 31/08, A61L 31/10, A61L 31/16, A61L 27/30, A61L 27/34, A61L 27/54

(54) **ANTIMIKROBIELLES MEDIZINTECHNISCHES PRODUKT, VERFAHREN ZU SEINER HERSTELLUNG UND VERWENDUNG**
ANTIMICROBIAL MEDICAL PRODUCT, METHOD FOR THE PRODUCTION THEREOF AND USE THEREOF
PRODUIT MEDICAL ANTIMICROBIEN, PROCEDE DE FABRICATION ET UTILISATION

(30) Priorität: 09.09.2005 DE 102005044360; 03.03.2006 DE 102006011217
(43) Veröffentlichungstag der Anmeldung: 10.10.2007
(73) Patentinhaber: Aesculap AG, 78532 Tuttlingen (DE); Albert-Ludwigs-Universität Freiburg, 79085 Freiburg (DE)
(72) Erfinder: HO, Chau Hon, 79106 Freiburg (DE); ODERMATT, Erich, K., CH-8200 Schaffhausen (CH); BARGON, Rainer, 88512 Mengen (DE); TILLER, Jörg, 79110 Freiburg (DE)
(74) Vertreter: Patentanwälte Ruff, Wilhelm, Beier, Dauster & Partner
(86) Internationale Anmeldenummer: PCT/EP2006/008714
(87) Internationale Veröffentlichungsnummer: WO 2007/028607

(56) Entgegenhaltungen:
- WO-A-2004/101011
- WO-A1-03/072143
- WO-A2-2004/056404
- WO-A2-2004/085998
- US-A1- 2004 171 978
- US-A1- 2005 130 240
- DATABASE WPI Week 2004 Derwent Publications Ltd., London, GB; AN 2004-157484 XP002422190 LI G, XIANG J, ZHU S: "Gene carrier for therapy of cerebral gliocyte diseases" & CN 1 456 356 A ((UYZH-N) UNIV ZHONGNAN) 19. November 2003 (2003-11-19)

## Beschreibung

Die Erfindung betrifft ein medizintechnisches Produkt mit einer antimikrobiellen Ausstattung, ein Verfahren zu dessen Herstellung sowie die Verwendung der antimikrobiellen Ausstattung als Biozid bei medizintechnischen Produkten.

Die in den letzten Jahren permanent ansteigenden Anforderungen an hygienische Standards führen insbesondere auf dem Gebiet der Medizin zu einem erheblichen Bedarf an antimikrobiellen Materialien. Da gewöhnliche Verbrauchsmaterialien, beispielsweise Holz, Keramik, Kunststoff, Glas oder Stahl selbst keine antimikrobiellen Eigenschaften besitzen, müssen sie antimikrobiell gemacht werden.

Ein leistungsstarker Ansatz hierzu basiert auf sogenannten kontaktaktiven Systemen, wobei Materialien derart mit einer antimikrobiellen Modifikation versehen werden, daß Mikroorganismen bei Kontakt mit dem modifizierten Material getötet werden, ohne im Gegensatz zu den ebenso gebräuchlichen Freisetzungssystemen eine nur begrenzt vorhandene antimikrobielle Verbindung freizusetzen. Kontakt-aktive Systeme bestehen häufig aus aufgepropften antimikrobiellen Polymeren, insbesondere polykationischen Polymeren mit Ammonium-, Pyridinium-, Biguanidin-, Sulfonium- oder Phosphoniumgruppen. Allerdings erfordert die Aufbringung der Polymere auf das betreffende Material nicht selten aufwendige Oberflächenmodifizierungen. So ist beispielsweise aus der US 2004/0171978 A1 bekannt, daß für die Immobilisierung von Polylysin auf eine Polymeroberfläche zuerst eine Sulfonierung der Oberfläche durchgeführt wird. Außerdem sind einige kontakt-aktive Systeme aufgrund von toxischen Eigenschaften mancher antimikrobieller Polymere für medizinische Anwendungen nur eingeschränkt einsatzfähig.

WO2004/056404 offenbart medizintechnische Gegenstände mit antimikrobiellen Beschichtungen, welche insbesondere Poly-ε-Lysin umfassen können.

WO03/072143 offenbart eine bioverträgliche Zusammensetzung zur Verabreichung eines Wirkstoffes. Die Zusammensetzung weist dabei einen Träger mit einer Bindungsdomäne für ein Metall, ein an die Bindungsdomäne komplexiertes Metallion sowie einen Wirkstoff auf. Als polymere Träger werden unter anderen diverse Polyaminosäuren aufgeführt.

WO2004/101011 offenbart biozides medizintechnisches Produkt enthaltend verzweigte amphiphile Makromoleküle Metallnanopartikel, Verfahren zu seiner Herstellung und Verwendung.

WO2004/085998 offenbart ein dreidimensionales Konstrukt auf der Basis einer Polymermatrix und von chemisch funktionalisierten Nanopartikeln. Die Nanopartikel sind dabei mit einer monomolekularen Schicht, welche eine biologische Information enthält, beschichtet.

Im medizinischen und klinischen Bereich bestehen jedoch hohe Anforderungen an die Bioverträglichkeit von Materialien, insbesondere von solchen, die für einen chirurgischen Einsatz bestimmt sind. Häufig verbleibt daher lediglich ein kleiner Spielraum zwischen antimikrobieller Wirksamkeit und Bioverträglichkeit des betreffenden Materials.

Aufgabe der vorliegenden Erfindung ist es daher, medizintechnische Produkte mit hoher antimikrobieller Wirksamkeit einerseits und hoher Bioverträglichkeit andererseits bereitzustellen.

Diese Aufgabe wird gelöst durch ein medizintechnisches Produkt mit einer antimikrobiellen bzw. bioziden Ausstattung aus einem Komplexmaterial aus Metallnanopartikeln und Makromolekülen, wobei die Makromoleküle mindestens teilweise aus einer Polyaminosäure gebildet werden, dadurch gekennzeichnet, dass die Polyaminosäure mit einer Substanz amphiphil modifiert ist und es sich bei der Substanz um eine Fettsäure oder ein Fettsäurederivat handelt.

Unter einer antimikrobiellen bzw. bioziden Ausstattung im Sinne der vorliegenden Erfindung soll eine Ausstattung verstanden werden, welche Zellwachstum und/oder -proliferation von Mikroorganismen, insbesondere von Keimen (schädliche Mikroorganismen), verhindert und/oder die Abtötung von vorhandenen Mikroorganismenkolonien, insbesondere Keimkolonien, bewirkt.

Gemäß einer Ausführungsform des medizintechnischen Produktes ist die antimikrobielle Ausstattung zumindest auf einem Teil der Oberfläche des Produktes, insbesondere in Form einer Beschichtung, vorgesehen. Die antimikrobielle Ausstattung ist vorzugsweise zumindest auf einem Teil der Oberfläche des Produktes, insbesondere in Form einer Beschichtung, vorhanden. Vorzugsweise erstreckt sich die antimikrobielle Ausstattung über die gesamte Oberfläche des medizintechnischen Produktes. Das derart ausgestattete medizintechnische Produkt zeichnet sich vorteilhafterweise dadurch aus, daß eine hinreichend stabile Haftverbindung zwischen der Ausstattung und der Oberfläche des Produktmaterials besteht, so daß beispielsweise ein Ablösen, insbesondere Abwischen oder Abwaschen, der Ausstattung vom beschichteten Produkt verhindert und somit eine mittelfristige, langfristige und wirkungsvolle Prävention des medizintechnischen Produktes gegen mikrobielle Besiedelung, insbesondere nach erfolgter Applikation, gewährleistet wird. Die Haftverbindung kann auf elektrostatischen Anziehungskräften, Wasserstoffbrückenbindungen und/oder lipophilen Interaktionen, insbesondere Van-der-Waals-Kräften, beruhen.

Zusätzlich oder alternativ zu der soeben beschriebenen Ausführungsform kann es erfindungsgemäß vorgesehen sein, daß sich das Komplexmaterial innerhalb des Produktes befindet. Dies kann besonders vorteilhaft sein, wenn es sich bei dem Werkstoff des medizintechnischen Produktes um ein Polymer oder auch ein anderes Material handelt, dessen Herstellungsprozess die Einführung des Komplexmaterials in das Innere des Produktes erlaubt. Auf diese Weise kann eine gleichmäßig verteilte antimikrobielle Wirksamkeit des medizintechnischen Produktes erzielt werden.

In einer weiteren Ausführungsform ist jeder Metallnanopartikel von mindestens einer Polyaminosäure umgeben, wobei jeder Metallnanopartikel vorzugsweise von allen Seiten hüllenartig von mindestens einer Polyaminosäure umgeben ist. Bevorzugt ist der polare, insbesondere geladene, Teil der Polyaminosäure zu den Metallnanopartikeln hin orientiert und ermöglicht durch die im polaren Teil befindlichen Heteroatome bzw. Heteroatomgruppierungen, beispielsweise Stickstoff- und/oder Sauerstoffatome, koordinative bzw. donative Bindungen zu den Metallnanopartikeln. Erfindungsgemäß können die Metallnanopartikel auf diese Weise partiell positiv polarisiert sein.

Die Polyaminosäure kann insbesondere eine Homo- oder HeteroPolyaminosäure sein, wobei eine Homo-Polyaminosäure besonders bevorzugt ist. Die Polyaminosäure kann aus natürlich vorkommenden und/oder synthetischen Aminosäuren bestehen, wobei natürlich vorkommende Aminosäuren, insbesondere α-Aminocarbonsäuren, insbesondere eine L-Konfiguration aufweisende α-Aminocarbonsäuren, bevorzugt sind. Bevorzugt handelt es ich bei den Aminosäuren um mindestens trifunktionelle Aminosäuren. Unter einer trifunktionellen Aminosäure im Sinne der vorliegenden Erfindung soll eine Aminosäure verstanden werden, welche zusätzlich zu der Carboxyl- und α-Aminogruppe eine weitere organische funktionelle Gruppe, insbesondere eine weitere Hydroxyl- Thiol-, Guanidin- oder Aminogruppe, vorzugsweise eine weitere Aminogruppe, aufweist. Vorzugsweise enthält die Polyaminosäure mindestens eine basische, saure und/oder schwefelhaltige Gruppe tragende Aminosäure. Bei der schwefelhaltigen Gruppe kann es sich insbesondere um Thiol- und/oder Thioethergruppen handeln. Besonders bevorzugt enthält die Polyaminosäure mindestens eine Aminosäure aus der Gruppe, umfassend Cystein, Methionin, Tryptophan, Histidin, Arginin, Lysin, Ornithin, Asparaginsäure, Glutaminsäure und deren Derivate.

Vorteilhafterweise weist die Polyaminosäure eine lineare Struktur auf. Die lineare Struktur erlaubt eine dichte Anordnung um die zu stabilisierenden Metallnanopartikel, wobei die Anordnung insbesondere durch elektrostatische Kräfte, Wasserstoffbrückenbindungen und/oder lipophile Wechselwirkungen, insbesondere Van-der-Waals-Kräfte, stabilisiert wird.

In einer anderen bevorzugten Ausführungsform der Erfindung weist die Polyaminosäure eine verzweigte, vorzugsweise eine hyperverzweigte, Struktur auf. Die verzweigte Struktur erlaubt insbesondere eine kompakte Anordnung um die zu stabilisierenden Metallnanopartikel, wodurch insbesondere die Stabilisierung der Metallnanopartikel erhöht wird. Weiterhin bewirkt die verzweigte Struktur der Polyaminosäure in vorteilhafter Weise eine verringerte Sprödigkeit des Komplexmaterials des erfindungsgemäßen Produktes. Somit erhöhen verzweigte Polyaminosäuren in bevorzugter Weise die filmbildenden Eigenschaften des Komplexmaterials.

Gemäß einer bevorzugten Ausführungsform des medizintechnischen Produktes ist die Polyaminosäure Polylysin, insbesondere Poly-α-Lysin (Poly-alpha-Lysin) und/oder Poly-ε-Lysin (Poly-epsilon-Lysin). Sowohl Poly-α-Lysin als auch Poly-e-Lysin weisen antimikrobielle Eigenschaften auf, wobei Poly-ε-Lysin im Gegensatz zu Poly-α-Lysin bioverträglicher und daher besonders bevorzugt ist. Das Polylysin des erfindungsgemäßen Produktes weist insbesondere einen Polymerisationsgrad (DP, Degree of Polymersation) von 10 bis 15, insbesondere 12 bis 14, vorzugsweise von ca. 13, auf. Das Molekulargewicht des Polylysins liegt vorzugsweise zwischen 3000 g/mol und 6000 g/mol, insbesondere zwischen 4000 g/mol und 5000 g/mol.

Gemäß einer bevorzugten Ausführungsform des medizintechnischen Produktes ist die Polyaminosäure mit einer Substanz, insbesondere mit einer solchen mit mindestens einem aliphatischen Rest, amphiphil modifiziert. Eine derartige Modifizierung kann besonders bevorzugt sein, um die Stabilisierung der Metallnanopartikel und die gegenseitige Abschirmung der die Nanopartikel koordinierenden Polyaminosäuren zu erhöhen. Auf diese Weise kann die Entstehung größerer Nanopartikel, insbesondere in Form von Aggregaten, verhindert werden. Weiterhin kann die Bildung von Aggregaten von Polyaminosäuren vermieden werden. Vorzugsweise ist der aliphatische Rest nach Modifizierung der Polyaminosäure insbesondere von den Metallnanopartikeln weg nach außen orientiert. Die so erhaltene Struktur aus Metallnanopartikeln und amphiphil modifizierten Polyaminosäuren kann als sogenannte Core-Shell-Struktur (Kern-Hülle-Partikel) bezeichnet werden, wobei die die Metallnanopartikel unmittelbar umgebenden Polyaminosäuren den Kern und die Substanz die Schale der Struktur darstellen. Unter Amphiphilität im Sinne der vorliegenden Erfindung soll die Eigenschaft einer Verbindung verstanden werden, welche aufgrund ihrer molekularen Struktur sowohl hydrophile als auch lipophile Eigenschaften aufweist. Ein Komplex mit einer Core-Shell-Struktur ist insbesondere aus der DE 103 23 597 A1 bekannt, die im Wesentlichen aus amphiphil modifiziertem Polyethylenimin besteht.

Bevorzugt weist der aliphatische Rest der Substanz 6 bis 22, insbesondere 12 bis 18, vorzugsweise 16 und/oder 18, Kohlenstoffatome auf. Bei dem aliphatischen Rest kann es sich um ein Alkyl-, Alkenyl und/oder einen Alkinylsubstituenten handeln, wobei Alkylsubstituenten, insbesondere unverzeigt, besonders bevorzugt sind. So erlauben Alkylsubstituenten, insbesondere langkettige und vorzugsweise unverzweigte Alkylsubstituenten, im Bereich der Schale der Core-Shell-Struktur eine dichtere bzw. kompaktere Anlagerung der Alkylketten aneinander, die im Wesentlichen auf lipophilen Wechselwirkungen, insbesondere auf Van-der-Waals-Kräften, beruht.

Vorzugsweise handelt es sich bei der Substanz um mindestens eine bioverträgliche Substanz, insbesondere eine Fettsäure oder ein Fettsäurederivat, vorzugsweise um Palmitin- und/oder Stearinsäure. Für die amphiphile Modifikation der Polyaminosäure kann es bevorzugt sein, daß die Fettsäure als Fettsäurederivat, insbesondere in einer aktivierten Form, vorzugsweise als Fettsäurechlorid, vorliegt. Weiterhin kann es erfindungsgemäß bevorzugt sein, daß die Substanz als Gemisch verschiedener Substanzen, insbesondere verschiedener Fettsäuren oder Fettsäurederivate, vorliegt. So ist insbesondere ein Gemisch aus Palmitin- und Stearinsäurechlorid, beispielsweise ein Gemisch aus 60 Gewichtsanteilen Stearinsäurechlorid und 40 Gewichtsanteilen Palmitinsäurechlorid, wegen seines im Vergleich zu den reinen Fettsäurechloriden niedrigeren Preises besonders bevorzugt.

Gemäß einer besonders bevorzugten Ausführungsform des erfindungsgemäßen Produktes beruht die amphiphile Modifikation der Polyaminosäure mit der Substanz auf kovalenten Bindungen, insbesondere auf Amidbindungen. Vorzugsweise sind die Amidbindungen aus den freien Aminogruppen der Polyaminosäure und Acylgruppen der Substanz gebildet. Im Falle des Poly-e-Lysins handelt es sich bei den freien Aminogruppen um die α-Aminogruppen der Polyaminosäure. Ausgehend von den reinen Lysin-Monomeren liegt der Anteil an freien Aminogruppen nach Herstellung der unmodifizierten Polyaminosäure gewöhnlich bei ca. 50%. Das medizintechnische Produkt zeichnet sich vorzugsweise dadurch aus, daß der Anteil an freien Aminogruppen der Polyaminosäure nach der amphiphilen Modifikation der Polyaminosäure zwischen 0,5 und weniger als 50%, insbesondere zwischen 10 und 40%, insbesondere zwischen 20 und 40%, vorzugsweise bei ca. 37%, liegt, bezogen auf die ursprüngliche Aminogruppen-Gesamtmenge der zur Herstellung der Polyaminosäure verwendeten Aminosäure-Monomere, vorzugsweise Lysin-Monomere.

Gemäß einer weiteren besonders bevorzugten Ausführungsform des medizintechnischen Produktes ist die Substanz über eine vernetzende Komponente, insbesondere eine polyfunktionelle. Carbonsäure, vorzugsweise Zitronensäure, mit der Polyaminosäure verbunden. Die Vernetzung beruht vorzugsweise auf der Bildung von kovalenten Bindungen, insbesondere Amidbindungen, wobei die Amidbindungen durch Kondensation zwischen den Aminogruppen der Polyaminosäure und Säuregruppen, insbesondere Carboxylgruppen, der vernetzenden Komponente, gebildet sind. Die Vernetzung der Polyaminosäure ist besonders vorteilhaft, da auf diese Weise nach der amphiphilen Modifikation der vernetzten Polyaminosäure geschlossene Core-Shell-Strukturen vorliegen und die funktionellen Gruppen, insbesondere Carboxylgruppen, der vernetzenden Komponente die Anzahl an möglichen Koordinationsstellen für die Metallnanopartikel im Polymer erhöhen. Auf diese Weise können die Komplexierungseigenschaften für die Metallnanopartikel verbessert werden. Weiterhin können durch die Vernetzung bestimmte Eigenschaften des bioziden Komplexmaterials, insbesondere dessen filmbildenden Eigenschaften, verbessert werden.

Bevorzugt liegt der Anteil an freien Aminogruppen der Polyaminosäure nach Vernetzung der Polyaminosäure, insbesondere mit Zitronensäure, zwischen 25 und weniger als 50%, insbesondere zwischen 30 und 45%, vorzugsweise zwischen 35 und 43%, bezogen auf die ursprüngliche Aminogruppen-Gesamtmenge der zur Herstellung der Polyaminosäure verwendeten Aminosäure-Monomere, vorzugsweise Lysin-Monomere. Vorzugsweise weist ein mit 5 mol-% Zitronensäure bezüglich der eingesetzten Lysin-Monomere vernetztes Polylysin, insbesondere Poly-ε-Lysin, einen Anteil an freien Aminogruppen von ca. 43% und ein mit 10 mol-% Zitronensäure bezüglich der eingesetzten Lysin-Monomere vernetztes Polylysin, insbesondere Poly-ε-Lysin, einen Anteil an freien Aminogruppen von ca. 35% auf, bezogen auf die ursprüngliche Aminogruppen-Gesamtmenge der zur Herstellung der Polyaminosäure verwendeten Lysin-Monomere.

In einer weiteren bevorzugten Ausführungsform liegt der Anteil an freien Aminogruppen der Polyaminosäure nach der amphiphilen Modifikation der vernetzten Polyaminosäure zwischen 15 und 35%, insbesondere zwischen 25 und 35%, vorzugsweise bei ca. 30%, bezogen auf die ursprüngliche Aminogruppen-Gesamtmenge der zur Herstellung der Polyaminosäure verwendeten Aminosäuren-Monomere, vorzugsweise Lysin-Monomere. In einer besonders bevorzugten Ausführungsform weist ein mit 5 mol-% Zitronensäure bezüglich der eingesetzten Lysin-Monomere vernetztes Polylysin, insbesondere Poly-ε-Lysin, nach der amphiphilen Modifikation einen Anteil an freien Aminogruppen von ca. 32% und ein mit 10 mol-% Zitronensäure bezüglich der eingesetzten Lysin-Monomere vernetztes Polylysin, insbesondere Poly-ε-Lysin, nach der amphiphilen Modifikation einen Anteil an freien Aminogruppen von ca. 26% auf, bezogen auf die ursprüngliche Aminogruppen-Gesamtmenge der zur Herstellung der Polyaminosäure verwendeten Lysin-Monomere.

Im Falle der Metallnanopartikel kann es sich erfindungsgemäß um Gold-, Silber-, Kupfer- oder Zinknanopartikel handeln, wobei Silbernanopartikel besonders bevorzugt sind. Mit Vorteil weisen die Metallnanopartikel einen Durchmesser von 0,5 bis 20 nm, insbesondere 1 bis 20 nm, vorzugsweise 1 bis 14 nm, auf.

Vorzugsweise weisen Nanosilberpartikel, die durch unvernetzte und mit einem Gemisch aus Palmitin- und Stearinsäurechlorid amphiphil modifizierte Polyaminosäuren, insbesondere Polylysin, vorzugsweise Poly-ε-Lysin, stabilisiert sind, einen Durchmesser von ca. 6 nm auf, insbesondere nach Reduktion mit Ascorbinsäure. In manchen Fällen kann es jedoch bevorzugt sein, daß die Nanosilberpartikel einen kleineren Durchmesser, insbesondere von ca. 4 nm, aufweisen. Dies ist beispielsweise durch eine Reduktion mit dem Reduktionsmittel LiBHEt₃ möglich.

Gemäß einer anderen bevorzugten Ausführungsform weisen Nanosilberpartikel, die durch vernetzte, insbesondere durch Zitronensäure vernetzte, und mit einem Gemisch aus Palmitin- und Stearinsäurechlorid amphiphil modifizierte Polyaminosäuren, insbesondere Polylysin, vorzugsweise Poly-ε-Lysin, stabilisiert sind, einen Durchmesser von ca. 10 nm (5 mol-% Zitronensäure bezüglich der eingesetzten Lysin-Monomere) oder ca. 8 nm (10 mol-% Zitronensäure bezüglich der eingesetzten Lysin-Monomere) auf. Dies kann beispielsweise durch Reduktion mit Ascorbinsäure erreicht werden.

In manchen Fällen kann es wünschenswert sein, dass die Metallnanopartikel, insbesondere Silbernanopartikel, des Komplexmaterials einen Durchmesser im Bereich von 2,5 bis 3,5 nm aufweisen. Dies kann beispielsweise durch Verwendung von Polylysin, vorzugsweise von Poly-ε-Lysin, erreicht werden. Bevorzugt ist das Polylysin mit Zitronensäure vernetzt und insbesondere mit einem Gemisch aus Plamitin- und Stearinsäurechlorid amphipil modifiziert. So können die Metallnanopartikel im Falle einer Stabilisierung durch Polylysin, welches mit 5 mol-% Zitronensäure bezüglich der eingesetzten Lysin-Monomere vernetzt ist, einen Durchmesser von ca. 3,1 nm aufweisen. Im Falle der Verwendung von Polylysin, welches mit 10 mol-% Zitronensäure bezüglich der eingesetzten Lysin-Monomere vernetzt ist, können die Metallnanopartikel einen Durchmesser von ca. 2,7 nm aufweisen.

Erfindungsgemäß kann es weiterhin vorgesehen sein, daß es sich bei dem medizintechnischen Produkt um ein temporäres oder dauerhaftes Implantat für den menschlichen oder tierischen Körper handelt. Hierbei handelt es sich bei den antimikrobiell ausgestatteten Implantaten vorzugsweise um Gelenkimplantate, Stents, Schrauben, Nägel und Platten. Die Implantate können aus Metallen oder Metalllegierungen bestehen. Als weitere Materialien für die Implantate kommen insbesondere Kunststoffe in Betracht. Die Implantate können beispielsweise zur Reparatur von Frakturen verwendet werden. Bei den Implantaten kann es sich weiterhin um Netze, vorzugsweise um Herniennetze, handeln. Als weitere bevorzugte Implante kommen insbesondere Gefäßprothesen, Membranen sowie Folien, beispielsweise zur Adhäsionsprophylaxe, in Betracht. Erfindungsgemäss ist es weiterhin bevorzugt, dass es sich bei den Implantaten um Inkontinenzbänder sowie allgemein um textile Implantate handeln kann. Als textile Implantate kommen insbesondere Gewebe, Gestricke, Gewirke, Gelege und Vliese in Frage. Die biozide Ausstattung dieser Implantate ermöglicht es, diese auch in akut infizierte oder infektions-gefährdete Körperregionen einzuführen, da die Implantate selbst durch die Ausstattung antimikrobiell wirken und auf diese Weise zu einer Verringerung einer vorhandenen oder potentiellen Infektion beitragen.

In einer anderen Ausführungsform handelt es sich bei den medizintechnischen Produkten um medizinische Instrumente, insbesondere um chirurgische Scheren, Zangen und Klammern sowie um Katheter oder Sonden und weitere Instrumente, insbesondere für minimalinvasive Eingriffe. In diesem Zusammenhang ist die bereits erwähnte Haftverbindung der antimikrobiellen Ausstattung mit der Oberfläche des medizintechnischen Produktes von besonderem Vorteil, da die beispielsweise soeben bezeichneten medizinischen Instrumente einer besonders hohen mechanischen Beanspruchung, insbesondere durch Reiben und Wischen, ausgesetzt sind. Die Haftung der antimikrobiellen Ausstattung mit der Produktoberfläche wird insbesondere durch lipophile Wechselwirkungen, vorzugsweise Van-der-Waals-Kräfte, der insbesondere von den Metallnanopartikeln wegweisenden langkettigen aliphatischen. Reste der Substanz mit der Produktoberfläche bewirkt.

Bei den medizintechnischen Produkten gemäß der vorliegenden Erfindung kann es sich ferner um Erzeugnisse wie beispielsweise Drainageschläuche, Nahtmaterialien oder Wundauflagen handeln. Bei dem Material des medizintechnischen Produktes handelt es sich gemäß einer weiteren bevorzugten Ausführungsform um ein Metall oder eine Metalllegierung, insbesondere um Titan, Edelstahl, Magnesium, Tantal oder einer Legierung davon, wobei Magnesium und/oder Tantal wegen ihrer Bioverträglichkeit und Resorbierbarkeit besonders bevorzugt sind.

In einer weiteren Ausführungsform handelt es sich bei dem Material des medizintechnischen Produktes um ein nichtresorbierbares oder um ein mindestens teilweise resorbierbares Polymer. So kann es sich bei dem nichtresorbierbaren Polymer um ein Polyolefin, insbesondere Polyethylen und/oder Polypropylen, einen Polyester, insbesondere Polyethylenterephthalat und/oder Polybutylenterephthalat, ein Polyamid, insbesondere Polyamid-6 oder Polyamid-6,6, oder eine Naturfaser, insbesondere Seide oder Leinen, handeln. Bevorzugt ist das mindestens teilweise resorbierbare Polymer ein vollständig resorbierbares Polymer. Bei dem resorbierbaren Polymer kann es sich insbesondere um ein Polymer auf der Basis der Monomere Lactid, Glykolid, Trimethylencarbonat, para-Dioxanon und/oder ε-Caprolacton, vorzugsweise in Form eines Co- und/oder Terpolymers, handeln. Entsprechend einer weiteren Ausführungsform kann ein medizintechnisches Produkt, dessen Material nicht resorbierbar ist, mit einem mindestens teilweise resorbierbaren, vorzugsweise vollständig resorbierbaren, Polymer, insbesondere mit einem der soeben aufgeführten Polymere, beschichtet werden, um somit die Zutrittsdauer von Flüssigkeiten, insbesondere von Körperflüssigkeiten, zu der antimikrobiellen Ausstattung zu beeinflußen bzw. zu regulieren.

In einer weiteren Ausführungsform handelt es sich bei dem Material des medizintechnischen Produktes um einen keramischen Werkstoff. Mit Vorteil kann es sich um einen resorbierbaren keramischen Werkstoff, insbesondere um Hydroxylapatit oder Tricalciumphosphat, handeln.

Gemäß einer weiteren Ausführungsform weist das Produkt Poren, vorzugsweise interkonnektierende Poren, auf. Dies kann besonders vorteilhaft sein, da auf diese Weise eine vergrößerte Oberfläche für die antimikrobielle Ausstattung zur Verfügung steht. Somit kann eine größere Menge des bioziden bzw. antimikrobiellen Komplexmaterials auf und, im Falle eines interkonnektierenden Porensystems, auch innerhalb des auszustattenden Produktes aufgebracht werden.

Das Produkt ist mit Vorteil sterilisierbar und liegt insbesondere in sterilisierter Form vor. Als Sterilisierungsmethoden kommen alle dem Fachmann bekannten Methoden, insbesondere Bestrahlung, Dampfsterilisation, Ethylenoxidbegasung und Plasmasterilisation in Frage, die vorzugsweise die chemische Struktur und/oder die antimikrobiellen Eigenschaften des insbesondere in Form einer Core-Shell-Struktur vorliegenden Komplexmaterials nicht beeinträchtigen. Das erfindungsgemäße medizintechnische Produkt liegt im Gebrauchszustand vorzugsweise in steriler Form vor.

Der Gegenstand der Erfindung betrifft außerdem ein Verfahren zur Herstellung eines medizintechnischen Produktes, wobei das Komplexmaterial, insbesondere in Form einer Lösung, von außen auf das nicht ausgestattete Produkt aufgebracht wird. In der Lösung liegen die Metallnanopartikel, insbesondere Silbernanopartikel, vorzugsweise gemäß einer der vorhergehenden Ausführungsformen stabilisiert vor. Die Herstellung einer derartigen Lösung erfolgt vorzugsweise ausgehend von amphiphil modifizierten und insbesondere vernetzten Polyaminosäuren. Die so hergestellten Core-Shell-Polymere werden in einem organischen Lösungsmittel aufgelöst und durch Zugabe eines Metallsalzes mit dem entsprechenden Metallion beladen. Die auf diese Weise hergestellte Lösung enthält durch amphiphil modifizierte und insbesondere vernetzte Polyaminosäuren stabilisierte Metallionen und eignet sich insbesondere für die antimikrobielle Ausstattung von medizintechnischen Produkten, insbesondere zur Ausstattung der bereits genannten medizintechnischen Produkte. Bevorzugt werden die derartig stabilisierten Metallionen der Lösung jedoch in Gegenwart eines geeigneten Reduktionsmittels, insbesondere Vitamin C, Natriumborhydrid, LiHBEt₃ oder einem Aldehyd, zu elementaren Metallnanopartikeln reduziert. Im Falle der Verwendung von LiHBEt₃ (Li: Lithium, H: Wasserstoff, B: Bor und Et: Ethyl) als Reduktionsmittel können davon abgeleitete Lithium-Bor-Spezies aufgrund der Oxophilie des Bors zu einer Vernetzung und damit zu einer Aggregation der amphiphil modifizierten und insbesondere vernetzten Polyaminosäuren führen. Daher und insbesondere wegen seiner Bioverträglichkeit ist die Verwendung von Vitamin C als Reduktionsmittel besonders bevorzugt. Als organische Lösungsmittel können diverse Alkohole, insbesondere Isopropanol oder Propanol, oder aromatische Lösungsmittel, beispielsweise Toluol oder Xylol, sowie Mischungen davon verwendet werden.

Weiterhin kann es bevorzugt sein, das anuimikrobielle Komplexmaterial als Feststoff, beispielsweise durch Sputtering, oder in Form einer Schmelze oder eines Aerosols auf das auszustattende Produkt aufzubringen.

Vorzugsweise wird das antimikrobielle Komplexmaterial in einem Tauchverfahren auf die Oberfläche des nicht ausgestatteten Produktes aufgebracht. Weiterhin kann das antimikrobielle Komplexmaterial durch Quellung in und/oder auf das nicht ausgestattete Produkt gebracht werden. Für die antimikrobielle Ausstattung von beispielsweise Nahtmaterialien, Netzen oder Bändern kann es bevorzugt sein, das biozide Komplexmaterial im Durchzugsverfahren von außen auf das nicht ausgestattete Produkt aufzubringen. Weiterhin kann das biozide Komplexmaterial durch dem Fachmann bekannte Ausgieß-, Ausstreich-, Präge- und Sprühtechniken, insbesondere Pressen, Walzen oder Rakeln, auf das nicht ausgestattete Produkt aufgebracht werden.

Gemäß einer weiteren Ausführungsform wird zusätzlich ein mindestens teilweise resorbierbares, vorzugsweise vollständig resorbierbares, Polymer, insbesondere in Form einer Lösung, auf die Oberfläche des Produktes aufgebracht. So kann es bevorzugt sein, daß das erfindungsgemäße Produkt nach einer oberflächlichen Beschichtung mit dem antimikrobiellen Komplexmaterial in einem zweiten Beschichtungsverfahren mit einer zweiten Schicht eines resorbierbaren Polymers, insbesondere eines Polyesters, Polyurethans oder Silikons, versehen wird. Vorzugsweise wird als zweite Schicht ein resorbierbares Co- und/oder Terpolymer, insbesondere auf der Basis von Lactid, Glykolid, Trimethylencarbonat, Polybutyrat, para-Dioxanon und/oder ε-Caprolacton, aufgebracht.

Als Lösungsmittel können Alkohole, aliphatische Ester, Ketone oder aromatische Lösungsmittel eingesetzt werden, wobei Ethylacetat besonders bevorzugt ist. Weiterhin ist es möglich, daß das resorbierbare Polymer nach einer Oberflächenbehandlung des medizintechnischen Produktes, insbesondere nach einer Plasmaaktivierung, auf das Produkt aufgebracht wird.

Alternativ dazu kann das mindestens teilweise resorbierbare, vorzugsweise vollständig resorbierbare, Polymer und das antimikrobielle Komplexmaterial gemeinsam in einem Beschichtungsprozess auf das auszustattende medizintechnische Produkt aufgebracht, werden. Dies ist besonders vorteilhaft, da ein einziger Beschichtungsprozess wirtschaftlicher und damit kostengünstiger ist.

Weiterhin ist es möglich, daß eine Keramik- und/oder Metallbeschichtung, insbesondere gemäß einer der beiden zuletzt beschriebenen Ausführungsformen, auf das auszustattende Produkt aufgebracht wird.

Gegenstand der Erfindung ist weiterhin ein Verfahren zur Herstellung eines medizintechnischen Produktes, wobei das Komplexmaterial, insbesondere in Form einer Lösung, zusätzlich oder alternativ zu den vorherigen Ausführungsformen in den Werkstoff des Produktes bei dessen Herstellung zugegeben oder nach Herstellung des Produktes durch Quellung in dieses eingelagert wird. Durch die Zugabe zum Werkstoff des Produktes kann eine gleichmäßige Verteilung des antimikrobiellen Komplexmaterials innerhalb des medizintechnischen Produktes sowie auf dessen Oberfläche oder zumindest in Schichten nahe der Produktoberfläche erreicht werden. Bezüglich weiterer Einzelheiten, insbesondere in Bezug auf die Lösung sowie auf alternative Zugabeformen des antimikrobiellen Komplexmaterials zum Werkstoff des medizintechnischen Produktes, insbesondere als Feststoff oder in Form einer Schmelze oder eines Aerosols, wird auf die obige Beschreibung verwiesen.

In einer weiteren vorteilhaften Ausführungsform wird das antimikrobielle Komplexmaterial mit dem Produktwerkstoff gemischt und anschließend zum gewünschten Produkt geformt, insbesondere extrudiert, gesponnen, gepreßt, gewalzt, gegossen oder geblasen. Besonders bevorzugt wird eine Mischung aus Polymer und antimikrobiellem Komplexmaterial zu einem Fadenmaterial versponnen, welches je nach Art des verwendeten Polymers zu resorbierbarem oder zu nicht resorbierbarem Nahtmaterial oder zu einem textilen Produkt verwebt oder verwirkt werden kann.

Die vorliegende Erfindung betrifft weiterhin ein Verfahren zur Herstellung von mindestens einer Polyaminosäuren, insbesondere zur Herstellung des erfindungsgemäßen Produktes, durch Polymerisation von mindestens trifunktionellen Aminosäuren in flüssiger Phase, wobei die Aminosäuren für die Polymerisation aktiviert und ohne Verwendung von Schutzgruppen polymerisiert werden.

In einer Ausführungsform des erfindungsgemäßen Verfahrens werden zur Bereitstellung der flüssigen Phase organische Lösungsmittel oder organische Lösungsmittelgemische verwendet. Mit Vorteil wird die Polymerisation der Aminosäuren in mindestens einem Lösungsmittel, ausgewählt aus der Gruppe, umfassend Dimethylsulfoxid (DMSO), Dimethylformamid (DMF), Dichlormethan, Tetrahydrofuran (THF) und Ethylacetat, vorgenommen.

In einer bevorzugten Ausführungsform des erfindungsgemäßen Verfahrens werden die Aminosäuren durch mindestens einen Stoff, vorzugsweise einen organischen Stoff, aktiviert. Mit besonderem Vorteil wird als Stoff eine stickstoffhaltige Verbindung verwendet. Bevorzugt werden die Aminosäure durch Reaktion mit dem Stoff in besonders reaktive Zwischenprodukte (Intermediate), insbesondere in Aktivester, überführt und somit aktiviert. Die auf diese Weise aktivierten Aminosäuren können mit nukleophilen Gruppen, insbesondere mit Aminogruppen, von anderen Aminosäuren reagieren. Dadurch kann die Polymerisation der Aminosäuren zu der Polyaminosäure unter besonders milden Reaktionsbedingungen, insbesondere bei Raumtemperatur, vorgenommen werden.

In einer besonderen Ausführungsform des erfindungsgemäßen Verfahrens werden die Aminosäuren durch mindestens einen Stoff aus der Gruppe, umfassend Carbodiimide, N-Hydroxysuccinimid (NHS), 1-Hydroxybenzotriazol (HOBT) und davon abgeleitete Derivate, aktiviert. Weiterhin können die Aminosäuren durch Reaktion mit Pentachlorophenol und/oder Pentafluorophenol aktiviert werden. Als Carbodiimide können insbesondere 1-Ethyl-3-(3-dimethylaminopropyl)-carbodiimid (EDC) oder Dicyclohexylcarbodiimid (DCC) verwendet werden. Die Verwendung von EDC kann insbesondere wegen seiner guten Wasserlöslichkeit bevorzugt sein. So kann beispielsweise das nach erfolgreicher Polymerisation entstandene und von EDC abgeleitete Isoharnstoffderivat durch einfache Reinigungsschritte, insbesondere durch eine wäßrige Extraktion, aus dem Reaktionsansatz entfernt werden. In einer anderen Ausführungsform kann es sinnvoll sein, die Aminosäuren für die Polymerisation durch DCC zu aktivieren.

In einigen Fällen ist das Aktivierungspotenzial von Carbodiimide, insbesondere von DCC, für eine zufriedenstellende Polymerisation nicht ausreichend, da beispielsweise die durch Carbodiimide aktivierte Zwischenprodukte insbesondere durch Folgereaktionen deaktiviert werden können. Bei diesen unerwünschten Folgereaktionen kann es sich insbesondere um Umlagerungsreaktionen handeln, wodurch die aktivierten Intermediate, insbesondere Aktivester, in deaktivierte Produkte, insbesondere Amidverbindungen, überführt werden, ehe sie zur gewünschten Polyaminosäure polymerisieren können. In solchen Fällen kann es erfindungsgemäß sinnvoll sein, die Aminosäuren durch Carbodiimide, insbesondere DCC, und einem weiteren Stoff zu aktivieren. Vorzugsweise reagiert der weitere Stoff mit den aus den Carbodiimiden und den Aminosäuren gebildeten aktivierten Zwischenprodukten unter Bildung von neuen, vorzugsweise reaktiveren, Zwischenprodukten, insbesondere unter Bildung von Aktivestern. Die auf diese Weise hergestellten aktiveren Zwischenprodukte reagieren besonders schnell mit den Aminogruppen der für die Polymerisation eingesetzten Aminosäuren. Auf diese Weise kann mit besonderem Vorteil eine Deaktivierung von aktiven Zwischenprodukten vermieden werden. Insbesondere ist somit die Herstellung einer Polyaminosäure, beispielsweise einer Polyaminosäure mit einem gewünschten DP (Degree Of Polymerisation), möglich.

In einer besonders bevorzugten Ausführungsform des erfindungsgemäßen Verfahrens werden die Aminosäuren durch Dicyclohexylcarbodiimid (DCC) und N-Hydroxysuccinimid (NHS) aktiviert.

In einer anderen bevorzugten Ausführungsform des erfindungsgemäßen Verfahrens werden die Aminosäuren durch Dicyclohexylcarbodiimid (DCC) und 1-Hydroxybenzotriazol (HOBT) aktiviert.

Weiterhin können die Aminosäuren durch Thionylchlorid (SOCl₂) in DMF als Lösungsmittel aktiviert werden, wobei die Aktivierung vorzugsweise über eine DMF-katalysierte Umsetzung mit Thionylchlorid (SOCl₂) vorgenommen wird.

Weiterhin können die Aminosäuren durch Erhitzen der flüssigen Phase aktiviert werden. Mit besonderem Vorteil wird durch das Erhitzen eine Kondensationsreaktion zwischen den Carboxyl- und Aminogruppen der Aminosäuren unter Abspaltung von Wasser und Ausbildung von AmidBindungen vorgenommen. Die Aktivierung wird insbesondere bei einer Temperatur (Reaktionstemperatur) zwischen 100 und 150 °C, insbesondere zwischen 110 und 140 °C, durchgeführt. Die Aktivierung und Verknüpfung der Aminosäure-Monomere wird insbesondere während eines Zeitraums von 8 bis 96 Stunden, vorzugsweise während ca. 96 Stunden, vorgenommen. Mit Vorteil kann die Reaktionsführung bei unterschiedlichen Temperaturen, insbesondere bei zwei unterschiedlichen Temperaturen, durchgeführt werden. Mit besonderem Vorteil kann die Polymerisation bei Temperaturen von ca. 140 °C und ca. 110 °C durchgeführt werden. Bevorzugt wird der Reaktionsansatz zunächst auf eine Temperatur von ca. 140 °C gebracht und während ca. 48 Stunden bei dieser Temperatur gehalten. Anschließend wird der Reaktionsansatz in bevorzugter Weise auf eine Temperatur von ca. 110 °C abgekühlt und während weiteren ca. 48 Stunden bei dieser Temperatur gehalten. Bevorzugt werden als Anfangskonzentrationen für die Aminosäuren Konzentrationen zwischen 2 und 20 mol/l, insbesondere zwischen 5 und 17 mol/l, vorzugsweise eine Konzentration von ca. 15 mol/l, verwendet.

Weiterhin kann es erfindungsgemäß bevorzugt sein, daß die Aminosäuren durch Silylierung, insbesondere durch Reaktion mit Hexamethyldisilazan (HMDS), aktiviert werden. Durch die Silylierung entstehen silylierte Aminosäuren, wobei die Heteroatome der Aminosäuren mindesten teilweise kovalent mit Silylgruppen, insbesondere mit Trimethylsilylgruppen, verbunden sind. Die auf diese Weise aktivierten Aminosäuren können zur Polyaminosäure polymerisiert werden.

Die hergestellten Polyaminosäuren können insbesondere durch Filtration und/oder Dialyse aufgereinigt werden. Bei kationischen, insbesondere polykationischen, Polyaminosäuren erfolgt die Aufreinigung mit besonderem Vorteil mit Hilfe der sogenannten CMC(Carboxymethylcellulose)-Methode. Bei der CMC-Methode bilden die Polyaminosäuren mit der in wäßrig-basischer Umgebung löslichen polyanionischen Carboxymethylcellulose vorzugsweise stabile unlösliche Aggregate. Diese Aggregate können durch Filtration von der wäßrigen Umgebung abgetrennt und insbesondere beliebig oft gewaschen werden. Somit können sämtliche Verunreinigungen beseitigt werden. Die Freisetzung der Polyaminosäuren aus den unlöslichen Aggregaten erfolgt vorzugsweise durch Ansäuern in wäßriger Umgebung, da auf diese Weise die protonierte Carboxymethylcellulose (CMC) unlöslich bleibt und die Polyaminosäuren in Lösung gehen. Gegebenenfalls kann bei Anwendung der CMC-Methode auf den Dialyseschritt zur Reinigung der Polyaminosäuren verzichtet werden. Durch geeignete Wahl der Parameter, insbesondere des pH-Wertes, der Konzentration, der Zugabegeschwindigkeit und der Art der verwendeten Carboxymethylcellulose, kann erreicht werden, daß einzelne Aminosäuren und Oligomere der Polyaminosäure keine Aggregate mit CMC bilden und mit Vorteil durch Waschschritte entfernt werden können.

In einer weiteren bevorzugten Ausführungsform des erfindungsgemäßen Verfahrens wird die Polyaminosäure in flüssiger Phase amphiphil, insbesondere in Gegenwart von Basen, modifiziert. Zur Herstellung der flüssigen Phase kommen insbesondere organische Lösungsmittel oder - gemische, insbesondere THF, sowie Wasser in Betracht. Bevorzugt werden als Basen Amine, insbesondere Triethylamin, verwendet. Erfindungsgemäß ist es besonders bevorzugt, daß für die amphiphile Modifikation der Polyaminosäure wasserlösliche Basen, insbesondere Alkalilaugen, vorzugsweise Natronlauge, verwendet werden. Bevorzugt wird die Polyaminosäure in flüssiger Phase, vorzugsweise in wäßriger Phase, gelöst. In einer bevorzugten Ausführungsform des erfindungsgemäßen Verfahrens wird zu der Lösung der zu modifizierenden Polyaminosäure eine Lösung der für die amphiphile Modifikation vorgesehenen Substanz hinzugegeben. Bevorzugt wird die Substanz in mindestens einem organischen Lösungsmittel, insbesondere THF, gelöst. Weiterhin wird zu der Lösung der zu modifizierenden Polyaminosäure eine Lösung, insbesondere eine wäßrige Lösung, der Base hinzugegeben. In einer besonders bevorzugten Ausführungsform des erfindungsgemäßen Verfahrens wird die Lösung der für die amphiphile Modifikation vorgesehenen Substanz und die Lösung der Base jeweils gleichzeitig zu der Lösung der Polyaminosäure hinzugegeben. Mit besonderem Vorteil kann die Hinzugabe durch Hinzutropfen erfolgen. Erfindungsgemäß ist es weiterhin besonders bevorzugt, daß als Substanz für die amphiphile Modifikation der Polyaminosäure mindestens eine Fettsäure, vorzugsweise Palmitin- und/oder Stearinsäure, oder ein Fettsäurederivat, vorzugsweise Palmitin- und/oder Stearinsäurechlorid, verwendet werden. Bezüglich weiterer Einzelheiten wird auf die bisherige Beschreibung verwiesen.

Vorzugsweise werden mit Hilfe des oben beschriebenen erfindungsgemäßen Verfahrens verzweigte, insbesondere hyperverzweigte, Polyaminosäuren, insbesondere Homopolyaminosäuren, vorzugsweise Poly-ε Lysin, hergestellt. Bezüglich weiterer Einzelheiten wird auf die bisherige Beschreibung verwiesen.

Weiterhin umfässt die Erfindung die Verwendung eines Komplexmaterials aus Metallnanopartikeln und Makromolekülen, wobei die Makromoleküle mindestens teilweise aus einer Polyaminosäure gebildet werden und insbesondere jeden Metallnanopartikel hüllenartig umgeben, als Biozid bei einem medizintechnischen Produkt. Bezüglich weiterer Merkmale und Einzelheiten wird auf die bisherige Beschreibung Bezug genommen.

Das erfindungsgemäße Produkt weist durch die molekulare Struktur und den Aufbau seiner Ausstattung bioverträgliche, insbesondere gewebeverträgliche, und gleichzeitig äußerst wirkungsvolle antimikrobielle bzw. biozide Eigenschaften auf. Die Zusammensetzung des Komplexmaterials aus körpereigenen Stoffen bzw. aus Stoffen, die aus körpereigenen Verbindungen aufgebaut sind, und/oder aus zumindest im wesentlichen körperverträglichen Stoffen gewährleistet die soeben angeführte Bioverträglichkeit des antimikrobiell ausgestatteten Produktes.

Die antimikrobiellen Eigenschaften der Ausstattung beruhen sowohl auf der bioziden Wirkung der Metallnanopartikel, insbesondere Silbernanopartikel, als auch auf der bioziden Wirkung der Polyaminosäure, insbesondere Poly-ε-Lysin. Die Zusammenführung dieser antimikrobiell wirkenden Stoffe in Form eines Komplexmaterials bedingt dessen hohe Wirksamkeit gegen insbesondere schädliche Mikroorganismen bzw. Keime. Mit besonderem Vorteil kann durch eine Core-Shell-Struktur des Komplexmaterials einerseits die Stabilisierung der Metallnanopartikel bewirkt werden und damit eine Ausfällung und unkontrollierbare Akkumulation des Metalls im Körper verhindert werden. Andererseits vermittelt die hydrophobe Schale (Shell) der Core-Shell-Struktur die Haftung der Ausstattung insbesondere auf der Produktoberfläche. Auf diese Weise kann das Risiko einer unkontrollierbaren oder auch einer kontinuierlichen Freisetzung des Metalls in die Umgebung, insbesondere in das umliegende Körpergewebe, verringert werden. Somit können in diesem Zusammenhang möglicherweise auftretende Nebenwirkungen abgeschwächt oder im wesentlichen verhindert werden.

Weitere Merkmale der Erfindung ergeben sich durch die nachfolgende Beschreibung von bevorzugten Ausführungsformen anhand von Beispielen. Hierbei können die einzelnen Merkmale der Erfindung allein oder in Kombination miteinander verwirklicht sein. Die beschriebenen Ausführungsformen dienen lediglich zur Erläuterung und zum besseren Verständnis der Erfindung und sind in keiner Weise einschränkend zu verstehen.

### Beispiel 1: Herstellung von E-Poly-L-Lysin

Zu einer Lösung aus 0.200 g L-Lysinhydrochlorid in 4 ml Wasser (dest.) wurden bei Raumtemperatur 0.627 g 1-Ethyl-3-[3-(dimethylamino)propyl] carbodiimidhydrochlorid in fünf Portionen zugegeben, wobei die Reaktionslösung zwischen zwei Zugaben 24 Stunden gerührt wurde. Nach Polymerisationsende wurde das Reaktionsgemisch gegen Wasser dialysiert (Dialysemembran mit einem Cut-off von 2000 g/mol) und anschließend das Polypeptid erhalten. (Ausbeute: 0,016 g [9 % ]).

### Beispiel 2: Polymerisation von L-Lysin durch Zugabe von DCC

Zu einer gerührten farblosen Suspension aus 200 mg L-Lysin (1.37 mmol, 1.0 Äquivalente) in 5 ml Ethylacetat wurden 566 mmg (2.74 mmol, 2.0 Äquivalente) Dicyclohexylcarbodiimid zugegeben. Die farblose Suspension (nicht milchig) wurde 4 Tage bei Raumtemperatur gerührt und anschließend durch Entfernung des Lösungsmittels bis zur Trockene eingeengt. Der entstandene Feststoff wurde mit 20 ml Wasser versetzt und die entstandene farblose Suspension zentrifugiert (10 Minuten bei 4.000 Umdrehungen pro Minute). Der abgetrennte farblose Feststoff wurde zweimal mit je 10 ml Wasser gewaschen. Die vereinigten wässrigen klaren Phasen wurden auf ca. 10 ml Lösung eingeengt, wobei eine Trübung eintrat. Die entstandene Suspension wurde filtriert und das Filtrat gegen Wasser dialysiert (MWCO 2000). Aus der Dialyse wurden ca. 1 mg farbloses Polymer erhalten.

### Beispiel 3: Polymerisation von L-Lysin durch Zugabe von DCC/NHS

Zu einer gerührten farblosen Suspension aus 200 mg L-Lysin (1.37 mmol, 1.0 Äquivalente) in 5 ml Ethylacetat wurden 566 mg (2.74 mmol, 2.0 Äquivalente) Dicyclohexylcarbodiimid und 316 mg (2.74 mmol, 2.0 Äquivalente) N-Hydroxysuccinimid (NHS) zugegeben. Die milchige Suspension wurde 4 Tage bei Raumtemperatur gerührt und anschließend durch Entfernung des Lösungsmittels bis zur Trockene eingeengt. Der entstandene Feststoff wurde mit 20 ml Wasser versetzt und die entstandene farblose Suspension zentrifugiert (10 Minuten bei 4.000 Umdrehungen pro Minute). Der abgetrennte farblose Feststoff wurde zweimal mit je 10 ml Wasser gewaschen. Die vereinigten wässrigen klaren Phasen wurden auf ca. 10 ml eingeengt, wobei eine Trübung eintrat. Die entstandene Suspension wurde filtriert und das Filtrat gegen Wasser dialysiert (MWCO 2000). Aus der Dialyse wurden 55 mg von farblosem Poly-L-Lysin (0.43 mmol Lysin-Einheiten, Ausbeute: 33 %) erhalten.

### Beispiel 4: Silylierung von L-Lysin mit Hexamethyldisilazan (HMDS)

Es wurden 9.14 g L-Lysinhydrochlorid (0.05 mol, 1.0 Äquivalente) in 53 ml Hexamethyldisilazan (41.02 g, 0.254 mmol, 5.1 Äquivalente) suspendiert und die Suspension auf 130 °C erhitzt. Nach 8 Stunden wurde eine gelbe Lösung erhalten. Nach 24 Stunden wurde die Lösung dunkelbraun. Es wurde restliches Hexamethyldisilazan im Vakuum (63 °C, 20 mbar) entfernt und eine dunkelbraune ölige Masse erhalten. Über Destillation (90 °C, 0.1 mbar) wurden 5.68 g farbloses Öl von silyliertem L-Lysin erhalten. Mit einem Silylierungsgrad von 1.9 pro Lysinmolekül berechnete sich eine Ausbeute von 40 % bezüglich des eingesetzten L-Lysins.

### Beispiel 5: Polymerisation des silylierten L-Lysins

Es wurden 2.860 mg des silylierten L-Lysins (ca. 0.012 mol Lysin-Anteil und ca. 0.023 mol, 1.0 Äquivalente, Trimethylsilyl-Anteil) unter Rückfluß auf 80 °C erhitzt und 1.0 ml Isopropanol (0.785 g, 0.013 mol, 0.6 Äquivalente) langsam dazugetropft. Es bildete sich langsam ein orangener Feststoff in einer hellgelben Flüssigkeit. Nach 8 Stunden wurde auf Raumtemperatur abgekühlt, und es wurden weitere 1.4 ml Isopropanol zugegeben. Nach einer Stunde wurden 2 ml Wasser zugegeben und das gesamte Lösungsmittel sowie das entstandene Hydroxytrimethylsilan im Vakuum entfernt. Der Rückstand wurde in Wasser gelöst und gegen Wasser dialysiert (MWCO 2000). Aus der Dialyse wurden 60 mg gelbliches Polymer (ca. 4 % Ausbeute) erhalten.

### Beispiel 6: Polykondensation

Es wurden 7.422 g L-Lysin *H₂O (0.045 mol) in 5.0 ml Wasser im Ultraschallbad gelöst. Anschließend wurde am Rotationsverdampfer so viel Wasser entfernt, bis eine Trübung eintrat, welche an Luft in eine klare dickflüssige Lösung überging. Durch Differenzwägung ergab sich, dass 3.0 ml Wasser als Lösungsmittel verblieben. Die wässrige L-Lysinlösung wurde auf 160 °C (Ölbadtemperatur) unter Rückfluss erhitzt und 2 Tage lang gerührt. Anschließend wurde auf 120 °C heruntertemperiert und weitere 2 Tage gerührt. Es wurde während der Reaktion regelmäßig ein leichter Stickstoffstrom für wenige Minuten durch die Apparatur geleitet, um Wasser zu entfernen. Nach beendeter Reaktion wurde auf Raumtemperatur abgekühlt und die sehr zähe orangene Masse in Wasser gelöst und anschließend gegen Wasser dialysiert (MWCO 2000). Aus der Dialyse wurden 2.278 g Polymer erhalten (Ausbeute: ca. 37 %).

### Beispiel 7: Modifizierung von ε-Poly-L-Lysin

Zu einer Suspension von 12 mg getrocknetem ε-Poly-L-Lysin in 3 ml i-Propanol (iso-Propanol) wurden bei Raumtemperatur 61 µl NEt₃ (Triethylamin) zugegeben. Der Ansatz wurde 30 Minuten gerührt. Anschließend wurde eine Lösung von 142 µl des Gemisches aus Palmitinund Stearinsäuresäurechlorid (Gemisch 40:60, Gew.-%) langsam in 1 ml i-Propanol zugetropft. Dabei wurde mehrmals für wenige Sekunden ein Vakuum angelegt (750-800 mbar), um gebildetes Aerosol zu entfernen. Nach vollendeter Zugabe des Säurechlorids wurde die Reaktionslösung weitere 24 Stunden bei Raumtemperatur gerührt. Anschließend wurden nochmals 61 µl NEt₃ zugegeben und 12 Stunden gerührt. Aus der Dialyse (Dialysemembran mit einem Cut-off von 2000 mg/mol) gegen i-Propanol wurde das mit Fettsäure modifizierte ε-Poly-L-Lysin erhalten (Modifizierungsgrad 27%, entspricht dem Anteil an freien Aminogruppen des Polylysins, die mit dem Gemisch aus Palmitin- und Stearinsäurechlorid reagiert haben).

### Beispiel 8: Modifizierung von Poly-L-Lysin

Es wurden 0.500 g des nach Beispiel 6 synthetisierten Polylysins (ca. 3.900 mmol, 1.0 Äquivalente) in 20 ml Wasser gelöst. Unter Rühren wurde bei Raumtemperatur eine Lösung aus 1.450 ml Säurechlorid (1.313 mg, 4.500 mmol, 1.2 Äquivalente) in 6 ml THF und gleichzeitig eine Lösung aus 0.180 g Natronlauge (4.500 mmol, 1.2 Äquivalente) in 2 ml Wasser zugetropft. Die Lösung wurde nach bereits Zugabe von geringen Mengen an Säurechloridlösung trübe. Nach beendeter Zugabe wurde eine milchige dicke Suspension erhalten, die über Nacht bei Raumtemperatur gerührt wurde. Die Suspension wurde zentrifugiert und anschließend filtriert und der farblose Feststoff zweimal mit je 30 ml Wasser gewaschen und anschließend im Vakuum getrocknet. Anschließend wurde i-Propanol zum Feststoff zugegeben und die gelbliche Suspension gegen i-Propanol dialysiert (MWCO 2000). Aus der Dialyse wurden 873 mg Polymer erhalten, d.h. bei vollständiger Umsetzung einer Ausbeute von 60 % des eingesetzten Poly-L-Lysin - nun amphiphil modifiziert.

### Beispiel 9: Vernetzung von ε-Poly-L-Lysin durch Zitronensäure

Es wurde zunächst eine Polymerisation von 0.200 g L-Lysinhydrochlorid (1.00 Äquivalente) gemäß Beispiel 1 durchgeführt. Anschließend wurde nach 24 Stunden der letzten EDC-Zugabe eine frisch zubereitete Lösung aus 0.011 g Zitronensäure (0.055 mmol, 0.05 Äquivalente) und 0.032 g EDC HCL (0.167 mmol, 0.15 Äquivalente) in 1 ml Wasser zugegeben. Für eine höhere Vernetzung wurden bei gleicher Vorgehensweise und gleichem Ansatz 0.021 g Zitronensäure (0.110 mmol, 0.10 Äquivalente) und 0.064 g EDC HCL (0.334 mmol, 0.30 Äquivalente) eingesetzt.

### Beispiel 10: Beladen der Polymerartikel mit Silber(I)-nitrat und Reduktion

Es wurden unter Stickstoffatmosphäre 10 mg modifiziertes ε-Poly-L-Lysin in 7,6 ml Toluol gelöst und 5,7 mg AgNO₃ in drei Portionen (24 Stunden Rührzeit nach jeder Zugabe) zugegeben. Es wurde eine klare und stabile Silber(I)-Polymer-Toluol-Lösung erhalten. Es wurden 0,1 ml der Silber(I)-Polymer-Toluol-Lösung (Inhalt: 0,13 mg Polymer, 0,05 mg AgNO₃) mit 2 ml i-Propanol verdünnt und mit 0,03 ml einer 0,02 M L-Ascorbinsäure-Lösung (in i-Propanol) versetzt. Die Lösung färbte sich intensiv gelb. Alternativ können 0,1 ml der Silber(I)-Polymer-ToluolLösung (inhalt: 0,13 mg Polymer, 0,05 mg AgNO₃) mit 2 ml i-Propanol verdünnt und mit 0,06 ml einer verdünnten 0,01 M (M: Molarität) Li[HBEt₃]-Lösung (in THF) versetzt werden. Die Lösung färbt sich ebenfalls intensiv gelb.

### Beispiel 11: Herstellung von Filmen auf Glasobjektträgern

Zur Herstellung eines Films wurde eine Silber(0)-Polymer-Lösung auf eine markierte Fläche von ca. 1 cm² eines Glasobjektträgers mit einer Pipette aufgetragen. Der Objektträger wurde auf eine Heizplatte gelegt und das Lösungsmittel abgedampft. Dabei wurde mit der Pipette die immer konzentrierter werdende Lösung auf dem Glas verrührt, so dass ein Film in der markierten Zone gebildet wurde.

### Beispiel 12: Bakterientests

Es wurden für die Bakterientests ca. 1 cm² große Filme auf Glasobjektträgern hergestellt: 4 µg Silber in 40 µg Polymer bzw. 10 µg Silber in 100 µg Polymer. Zur Vorbereitung der Bakterienzellen wurden 50 ml eines sterile Standard-Kulturmediums der Fa. Merck mit 100 µl Suspension von Staphylokokkus Aureus-Zellen (ca. 10¹¹ Zellen pro ml) in PBS (Phosphate buffered saline, pH 7.0) angeimpft und für 6 Stunden bei 37 °C unter Schütteln inkubiert. Nach Zentrifugieren der Bakteriensuspension wurden die Zellen zweimal mit PBS (pH 7.0) gewaschen, dann mit PBS resuspendiert und weiter mit PBS auf eine Konzentration von 5 x 10⁸ Zellen pro ml verdünnt. Die Zellkonzentration wurde durch die Absorption bei 600 nm überprüft. Die hergestellten Filme wurden für 2 Minuten in PBS gewaschen und mit der Bakteriensuspension besprüht. Anschließend wurden die Objektträger in je eine Petri-Schale gelegt und 25 ml an Wachstumsagar (1.5 Gew.% Agar in Wachstums medium wurde für 5 Minuten auf 100 °C erhitzt und schnell auf 40 °C abgekühlt) zugegeben. Danach wurden die Petri-Schalen bei 37 °C inkubiert. Die Filme mit einer Mindestmenge an Silber von 10 µg pro cm² verhinderten das Wachstum der aufgesprühten Staphylokokkus Aureus-Zellen zu mehr als 99 %.

## Patentansprüche

1. Medizintechnisches Produkt mit einer antimikrobiellen Ausstattung aus einem Komplexmaterial aus Metallnanopartikeln und Makromolekülen, wobei die Makromoleküle mindestens teilweise aus einer Polyaminosäure gebildet werden, **dadurch gekennzeichnet, dass** die Polyaminosäure mit einer Substanz amphiphil modifiziert ist und es sich bei der Substanz um eine Fettsäure oder ein Fettsäurederivat handelt.

2. Medizintechnisches Produkt nach Anspruch 1, **dadurch gekennzeichnet, dass** die Ausstattung zumindest auf einem Teil der Oberfläche des Produktes, insbesondere in Form einer Beschichtung, vorgesehen ist.

3. Medizintechnisches Produkt nach Anspruch 1 oder 2, **dadurch gekennzeichnet, dass** die Ausstattung im Inneren des Produktes vorgesehen ist.

4. Medizintechnisches Produkt nach einem der vorhergehenden Ansprüche, **dadurch gekennzeichnet, dass** jeder Metallnanopartikel hüllenartig von mindestens einer Polyaminosäure umgeben ist.

5. Medizintechnisches Produkt nach einem der vorhergehenden Ansprüche, **dadurch gekennzeichnet, dass** die Polyaminosäure eine Homo-oder Heteropolyaminosäure, insbesondere eine Homopolyaminosäure, ist.

6. Medizintechnisches Produkt nach einem der vorhergehenden Ansprüche, **dadurch gekennzeichnet, dass** die Polyaminosäure aus natürlich vorkommenden und/oder synthetischen, insbesondere aus natürlich vorkommenden, Aminosäuren besteht.

7. Medizintechnisches Produkt nach einem der vorhergehenden Ansprüche, **dadurch gekennzeichnet, dass** die Polyaminosäure mindestens eine basische, saure oder schwefelhaltige Aminosäure, insbesondere mindestens eine Aminosäure aus der Gruppe, umfassend Cystein, Methionin, Tryptophan, Histidin, Arginin, Lysin, Ornithin, Asparaginsäure, Glutaminsäure und deren Derivate, enthält.

8. Medizintechnisches Produkt nach einem der vorhergehenden Ansprüche, **dadurch gekennzeichnet, dass** die Polyaminosäure eine lineare Struktur aufweist.

9. Medizintechnisches Produkt nach einem der Ansprüche 1 bis 7, **dadurch gekennzeichnet, dass** die Polyaminosäure eine verzweigte, vorzugsweise eine hyperverzweigte, Struktur aufweist.

10. Medizintechnisches Produkt nach einem der vorhergehenden Ansprüche, **dadurch gekennzeichnet, dass** die Polyaminosäure Polylysin, insbesondere Poly-α-Lysin und/oder Poly-ε-Lysin, vorzugsweise Poly-ε-Lysin, ist.

11. Medizintechnisches Produkt nach Anspruch 10, **dadurch gekennzeichnet, dass** das Polylysin einen Polymerisationsgrad (DP, Degree Of Polymerisation) von 10 bis 15, insbesondere 12 bis 14, vorzugsweise von ca. 13, aufweist.

12. Medizintechnisches Produkt nach Anspruch 10 oder 11, **dadurch gekennzeichnet, dass** das Polylysin ein Molekulargewicht zwischen 3.000 und 6.000 g/mol, insbesondere zwischen 4.000 und 5.000 g/mol, aufweist.

13. Medizintechnisches Produkt nach einem der vorhergehenden Ansprüche, **dadurch gekennzeichnet, dass** es sich bei der Substanz um Palmitin- und/oder Stearinsäure handelt.

14. Medizintechnisches Produkt nach einem der vorhergehenden Ansprüche, **dadurch gekennzeichnet, dass** die amphiphile Modifikation der Polyaminosäure auf kovalenten Bindungen, insbesondere auf Amidbindungen, beruht.

15. Medizintechnisches Produkt nach einem der vorhergehenden Ansprüche, **dadurch gekennzeichnet, dass** der Anteil an freien Aminogruppen nach der amphiphilen Modifikation zwischen 0,5 und weniger als 50%, insbesondere zwischen 10 und 40%, insbesondere zwischen 20 und 40%, vorzugsweise bei ca. 37%, liegt, bezogen auf die ursprüngliche Aminogruppen-Gesamtmenge der zur Herstellung der Polyaminosäure verwendeten Aminosäure-Monomere, vorzugsweise Lysin-Monomere.

16. Medizintechnisches Produkt nach einem der vorhergehenden Ansprüche, **dadurch gekennzeichnet, dass** die Substanz über eine vernetzende Komponente, insbesondere eine polyfunktionelle Carbonsäure, vorzugsweise Zitronensäure, mit der Polyaminosäure verbunden ist, insbesondere kovalent.

17. Medizintechnisches Produkt nach einem der vorhergehenden Ansprüche, **dadurch gekennzeichnet, dass** der Anteil an freien Aminogruppen nach der amphiphilen Modifikation der vernetzten Polyaminosäure zwischen 15 und 45%, insbesondere zwischen 25 und 35%, vorzugsweise bei ca. 30%, liegt, bezogen auf die ursprüngliche Aminogruppen-Gesamtmenge der zur Herstellung der Polyaminosäure verwendeten Aminosäure-Monomere, vorzugsweise Lysin-Monomere.

18. Medizintechnisches Produkt nach einem der vorhergehenden Ansprüche, **dadurch gekennzeichnet, dass** es sich bei den Metallnanopartikeln um Gold-, Silber-, Kupfer oder Zinknanopartikel handelt, wobei Silbernanopartikel bevorzugt sind.

19. Medizintechnisches Produkt nach einem der vorhergehenden Ansprüche, **dadurch gekennzeichnet, dass** die Metallnanopartikel einen Durchmesser von 0,5 bis 20 nm, insbesondere 1 bis 20 nm, vorzugsweise 1 bis 14 nm, aufweisen.

20. Verfahren zur Herstellung eines medizintechnischen Produktes nach einem der Ansprüche 1 bis 19, **dadurch gekennzeichnet, dass** das Komplexmaterial, insbesondere in Form einer Lösung, von außen auf das nicht ausgestattete Produkt aufgebracht oder in den Werkstoff des Produktes bei dessen Herstellung zugegeben wird.

21. Verfahren zur Herstellung von mindestens einer Polyaminosäure, insbesondere zur Herstellung eines medizintechnischen Produktes nach einem der Ansprüche 1 bis 19, durch Polymerisation von mindestens trifunktionellen Aminosäuren in flüssiger Phase, **dadurch gekennzeichnet, daß** die Aminosäuren für die Polymerisa-tion aktiviert und ohne Verwendung von Schutzgruppen polymerisiert werden.

22. Verfahren nach Anspruch 21, **dadurch gekennzeichnet, dass** die Aminosäuren durch mindestens einen Stoff, vorzugsweise einen organischen Stoff, aktiviert werden.

23. Verfahren nach Anspruch 21 oder 22, **dadurch gekennzeichnet, dass** die Aminosäuren durch mindestens einen Stoff aus der Gruppe, umfassend Carbodiimide, N-Hydroxysuccinimid (NHS), 1-Hydroxybenzotriazol (HOBT), Pentafluorophenol, Pentachlorophenol und davon abgeleitete Derivate, aktiviert werden.

24. Verfahren nach einem der Ansprüche 21 bis 23, **dadurch gekennzeichnet, dass** die Aminosäuren durch Dicyclohexylcarbodiimid (DCC) und N-Hydroxysuccinimid (NHS) aktiviert werden.

25. Verfahren nach einem der Ansprüche 21 bis 24, **dadurch gekennzeichnet, daß** die Aminosäuren durch Dicyclohexylcarbodiimid (DCC) und 1-Hydroxybenzotriazol (HOBT) aktiviert werden.

26. Verfahren nach einem der Ansprüche 21 bis 25, **dadurch gekennzeichnet, dass** die Polyaminosäure amphiphil modifiziert wird, insbesondere in Gegenwart von Basen.

27. Verwendung eines Komplexmaterials aus Metallnanopartikeln und Makromolekülen als Biozid bei einem medizintechnischen Produkt, insbesondere bei einem solchen nach einem der Ansprüche 1 bis 19, wobei die Makromoleküle mindestens teilweise aus einer Polyaminosäure gebildet werden, **dadurch gekennzeichnet, dass** die Polyaminosäure mit einer Substanz amphiphil modifiziert ist und es sich bei der Substanz um eine Fettsäure oder ein Fettsäurederivat handelt.

## Claims

1. A medical product provided with an antimicrobial finishing comprised of a complex material made of metal nanoparticles and macromolecules, the macromolecules being at least partially formed from a poly(amino acid), **characterized in that** the poly(amino acid) is modified to be amphiphilic by a substance, and where the substance is a fatty acid or a fatty acid derivative.

2. The medical product according to claim 1, **characterized in that** the finishing is provided at least on part of a surface of the product, in particular in the form of a coating.

3. The medical product according to claim 1 or 2, **characterized in that** the finishing is provided inside the product.

4. The medical product according to any of the preceding claims, **characterized in that** each metal nanoparticle is enveloped by at least one poly(amino acid).

5. The medical product according to any of the preceding claims, **characterized in that** the poly(amino acid) is a homo- or heteropoly(amino acid), in particular a homopoly(amino acid).

6. The medical product according to any of the preceding claims, **characterized in that** the poly(amino acid) is comprised of naturally occurring and/or synthetic, in particular naturally occurring poly(amino acids).

7. The medical product according to any of the preceding claims, **characterized in that** the poly(amino acid) includes at least one basic, acid, or sulfurous amino acid, in particular at least one amino acid from the group consisting of cysteine, methionine, tryptophan, histidine, arginine, lysine, ornithine, aspartic acid, glutamic acid, and derivatives thereof.

8. The medical product according to any of the preceding claims, **characterized in that** the poly(amino acid) has a linear structure.

9. The medical product according to any of the claims 1 through 7, **characterized in that** the poly(amino acid) has a branched, preferably hyperbranched structure.

10. The medical product according to any of the preceding claims, **characterized in that** the poly(amino acid) is polylysine, in particular poly-α-lysine and/or poly-ε-lysine, preferably poly-ε-lysine.

11. The medical product according to claim 10, **characterized in that** the polylysine has a degree of polymerization (DP) from 10 to 15, in particular from 12 to 14, preferably of about 13.

12. The medical product according to claim 10 or 11, **characterized in that** the polylysine has a molecular weight from between 3000 to 6000 g/mol, in particular from between 4000 to 5000 g/mol.

13. The medical product according to any of the preceding claims, **characterized in that** the substance is palmitic and/or stearic acid.

14. The medical product according to any of the preceding claims, **characterized in that** the amphiphilic modification of the poly(amino acid) is based on covalent bonding, in particular amide bonding.

15. The medical product according to any of the preceding claims, **characterized in that** the proportion of free amine groups after the amphiphilic modification is from between 0.5 to less than 50 %, in particular from between 10 to 40 %, preferably about 37 %, based on the original total amount of amine groups of the amino acid monomers, in particular lysine monomers, used in production of the poly(amino acid).

16. The medical product according to any of the preceding claims, **characterized in that** the substance is bonded to the poly(amino acid), in particular covalently bonded via a cross-linking component, in particular a polyfunctional carboxylic acid, preferably citric acid.

17. The medical product according to any of the preceding claims, **characterized in that** the proportion of free amine groups after the amphiphilic modification of the cross-linked poly(amino acid) is from between 15 to 45 %, in particular from between 25 to 35 %, preferably about 30 %, based on the original total amount of amine groups of the amino acid monomers, in particular lysine monomers, used in production of the poly(amino acid).

18. The medical product according to any of the preceding claims, **characterized in that** the metal nanoparticles are made of gold, silver, copper or zinc nanoparticles, and silver nanoparticles being preferred.

19. The medical product according to any of the preceding claims, **characterized in that** the metal nanoparticles have a diameter from 0.5 to 20 nm, in particular from 1 to 20 nm, preferably from 1 to 14 nm.

20. A method for the production of a medical product according to any of the claims 1 through 19, **characterized in that** the complex material, in particular in the form of a solution, is applied on the exterior of the non-finished product or added to the material of the product during its production.

21. A method for the production of at least one poly(amino acid), in particular for the production of a medical product according to any of the claims 1 through 19, using polymerization of at least trifunctional amino acids in liquid phase, **characterized in that** the amino acids are activated for polymerization and are polymerized without use of protecting groups.

22. The method according to claim 21, **characterized in that** the amino acids are activated by at least one agent, preferably an organic agent.

23. The method according to claim 21 or 22, **characterized in that** the amino acids are activated by at least one agent from the group consisting of carbodiimides, N-hydroxysuccinimide (NHS), 1-hydroxybenzotriazole (HOBT), pentafluorophenole, pentachlorophenole, and derivatives thereof.

24. The method according to any of the claims 21 through 23, **characterized in that** the amino acids are activated by dicyclohexyl carbodiimide (DCC) and N-hydroxysuccinimide (NHS).

25. The method according to any of the claims 21 through 24, **characterized in that** the amino acids are activated by dicyclohexyl carbodiimide (DCC) and 1-hydroxybenzotriazole (HOBT).

26. The method according to any of the claims 21 through 25, **characterized in that** the amino acid is subject to amphiphilic modification, in particular in the presence of bases.

27. Use of a complex material made of metal nanoparticles and macromolecules as a biocide with a medical product, in particular a product according to any of the claims 1 through 19, wherein the macromolecules are at least partially formed of a poly(amino acid), **characterized in that** the poly(amino acid) is modified to be amphiphilic by a substance, and where the substance is a fatty acid or a fatty acid derivative.

## Revendications

1. Produit médical muni d'un garnissage antimicrobien constitué d'un matériau complexe à base de nanoparticules métalliques et de macromolécules, les macromolécules étant constituées au moins en partie d'un poly(acide aminé), **caractérisé en ce que** le poly(acide aminé) est modifié pour être amphiphile par une substance et la substance consiste en un acide gras ou un dérivé d'acide gras.

2. Produit médical selon la revendication 1, **caractérisé en ce que** le garnissage est prévu au moins sur une partie de la surface du produit, en particulier sous forme d'un revêtement.

3. Produit médical selon la revendication 1 ou 2, **caractérisé en ce que** le garnissage est prévu à l'intérieur du produit.

4. Produit médical selon l'une quelconque des revendications précédentes, **caractérisé en ce que** chaque nanoparticule métallique est entourée comme par une enveloppe d'au moins un poly(acide aminé).

5. Produit médical selon l'une quelconque des revendications précédentes, **caractérisé en ce que** le poly(acide aminé) est un homo- ou hétéropoly(acide aminé) en particulier un homopoly(acide aminé).

6. Produit médical selon l'une quelconque des revendications précédentes, **caractérisé en ce que** le poly(acide aminé) est constitué d'acides aminés existant dans la nature et/ou synthétiques, en particulier d'acides aminés existant dans la nature.

7. Produit médical selon l'une quelconque des revendications précédentes, **caractérisé en ce que** le poly(acide aminé) contient au moins un acide aminé basique, acide ou soufré, en particulier au moins un acide aminé choisi dans le groupe comprenant la cystéine, la méthionine, le tryptophane, l'histidine, l'arginine, la lysine, l'ornithine, l'acide aspartique, l'acide glutamique et leurs dérivés.

8. Produit médical selon l'une quelconque des revendications précédentes, **caractérisé en ce que** le poly(acide aminé) présente une structure linéaire.

9. Produit médical selon l'une quelconque des revendications 1 à 7, **caractérisé en ce que** le poly(acide aminé) présente une structure ramifiée, de préférence une structure hyper-ramifiée.

10. Produit médical selon l'une quelconque des revendications précédentes, **caractérisé en ce que** le poly(acide aminé) est la polysine, en particulier la poly-α-lysine et/ou la poly-ε-lysine, de préférence la poly-ε-lysine.

11. Produit médical selon la revendication 10, **caractérisé en ce que** la polylysine présente un degré de polymérisation (DP, *Degree of Polymerisation*) de 10 à 15, en particulier de 12 à 14, de préférence d'environ 13.

12. Produit médical selon la revendication 10 ou 11, **caractérisé en ce que** la polylysine a une masse moléculaire comprise entre 3 000 et 6 000 g/mole, en particulier entre 4 000 et 5 000 g/mole.

13. Produit médical selon l'une quelconque des revendications précédentes, **caractérisé en ce que** la substance consiste en l'acide palmitique et/ou l'acide stéarique.

14. Produit médical selon l'une quelconque des revendications précédentes, **caractérisé en ce que** la modification amphiphile du poly(acide aminé) repose sur des liaisons covalentes, en particulier sur des liaisons amide.

15. Produit médical selon l'une quelconque des revendications précédentes, **caractérisé en ce que** la teneur en groupes amino libres après la modification amphiphile est comprise entre 0,5 et moins de 50 %, en particulier entre 10 et 40 %, en particulier entre 20 et 40 %, de préférence est d'environ 37 %, par rapport à la quantité totale initiale de groupes amino des monomères acide aminé utilisés pour la préparation du poly(acide aminé), de préférence des monomères lysine.

16. Produit médical selon l'une quelconque des revendications précédentes, **caractérisé en ce que** la substance est liée, en particulier par liaison covalente, au poly(acide aminé) par l'intermédiaire d'un composant réticulant, en particulier un acide carboxylique polyfonctionnel, de préférence acide citrique.

17. Produit médical selon l'une quelconque des revendications précédentes, **caractérisé en ce que** la teneur en groupes amino libres après la modification amphiphile du poly(acide aminé) réticulé est comprise entre 15 et 45 %, en particulier entre 25 et 35 %, de préférence est d'environ 30 %, par rapport à la quantité totale initiale de groupes amino des monomères acide aminé utilisés pour la préparation du poly(acide aminé), de préférence des monomères lysine.

18. Produit médical selon l'une quelconque des revendications précédentes, **caractérisé en ce que** les nanoparticules métalliques consistent en des nanoparticules d'or, d'argent, de cuivre ou de zinc, les nanoparticules d'argent étant préférées.

19. Produit médical selon l'une quelconque des revendications précédentes, **caractérisé en ce que** les nanoparticules métalliques ont un diamètre de 0,5 à 20 nm, en particulier de 1 à 20 nm, de préférence de 1 à 14 nm.

20. Procédé pour la préparation d'un produit médical selon l'une quelconque des revendications 1 à 19, **caractérisé en ce que** le matériau complexe est appliqué, en particulier sous forme d'une solution, de l'extérieur, sur le produit non garni ou est ajouté dans le matériau du produit lors de sa préparation.

21. Procédé pour la préparation d'au moins un poly(acide aminé), en particulier destiné à la préparation d'un produit médical selon l'une quelconque des revendications 1 à 19, par polymérisation d'acides aminés au moins trifonctionnels en phase liquide, **caractérisé en ce que** les acides aminés sont activés pour la polymérisation et polymérisés sans utilisation de groupes protecteurs.

22. Procédé selon la revendication 21, **caractérisé en ce que** les acides aminés sont activés par au moins une substance, de préférence une substance organique.

23. Procédé selon la revendication 21 ou 22, **caractérisé en ce que** les acides aminés sont activés par au moins une substance choisie dans le groupe comprenant des carbodiimides, le N-hydroxysuccinimide (NHS), le 1-hydroxybenzotriazole (HOBT), le pentafluorophénol, le pentachlorophénol et des dérivés issus de ceux-ci.

24. Procédé selon l'une quelconque des revendications 21 à 23, **caractérisé en ce que** les acides aminés sont activés par le dicyclohexylcarbodiimide (DCC) et le N-hydroxysuccinimide (NHS).

25. Procédé selon l'une quelconque des revendications 21 à 24, **caractérisé en ce que** les acides aminés sont activés par le dicyclohexylcarbodiimide (DCC) et le 1-hydroxybenzotriazole (HOBT).

26. Procédé selon l'une quelconque des revendications 21 à 25, **caractérisé en ce que** le poly(acide aminé) est modifié pour être amphiphile, en particulier en présence de bases.

27. Utilisation d'un matériau complexe constitué de nanoparticules métalliques et de macromolécules, en tant que biocide dans un produit médical, en particulier dans un tel produit selon l'une quelconque des revendications 1 à 19, dans lequel les macromolécules sont constituées au moins en partie d'un poly(acide aminé), **caractérisée en ce que** le poly(acide aminé) est modifié pour être amphiphile par une substance et la substance consiste en un acide gras ou un dérivé d'acide gras.
